# EUROPÄISCHE PATENTSCHRIFT

(11) **EP 0 980 686 B1**
(45) Veröffentlichungstag und Bekanntmachung des Hinweises auf die Patenterteilung: **25.07.2007**
(21) Anmeldenummer: 99115512.8
(22) Anmeldetag: 05.08.1999
(51) Int. Cl.: A61M 1/36

(54) **Multifunktionssensor**
Multifunctional sensor
Capteur à fonctions multiples

(30) Priorität: 19.08.1998 DE 19837667
(43) Veröffentlichungstag der Anmeldung: 23.02.2000
(73) Patentinhaber: Fresenius Medical Care Deutschland GmbH, 61352 Bad Homburg v.d.H. (DE)
(72) Erfinder: Jahn, Paul, 60437 Frankfurt (DE); Koerdt, Franz Wilhelm, 61231 Bad Nauheim (DE); Krämer, Matthias, Dr., 61381 Friedrichsdorf (DE); Metzner, Klaus, 61381 Friedrichsdorf (DE); Peter, Harald, 61440 Oberursel (DE); Pieper, Walter, 61197 Florstadt (DE); Steinbach, Bernd, Dr., 61169 Friedberg (DE)
(74) Vertreter: Laufhütte, Dieter

(56) Entgegenhaltungen:
- EP-A- 0 130 441
- EP-A- 0 374 858
- DE-A- 4 419 593
- US-A- 4 072 056
- US-A- 5 563 347

## Beschreibung

Die Erfindung betrifft eine Vorrichtung zur Messung von Parametern medizinischer Flüssigkeiten nach dem Oberbegriff des Anspruchs 1.

Eine derartige Vorrichtung wird beispielsweise bei der Blutreinigung bzw. bei der Dialysebehandlung benötigt. Zur Überwachung der Dialysebehandlung ist sowohl eine Lufterkennung, als auch eine Drucküberwachung im arteriellen und venösen Teil des extrakorporalen Blutkreislaufs erforderlich. Weiterhin sind zur Verbesserung der Qualität der Dialysebehandlung die Messung von Blutvolumen und Bluttemperatur von Vorteil.

Zur Messung dieser Parameter sind verschiedene Vorrichtungen bekannt.

Aus der DE 38 27 553 C1 ist eine Vorrichtung zum Messen der Änderung des intravasalen Blutvolumens während der Blutfiltration in einer Blutreinigungseinrichtung bekannt. Hierzu ist im extrakorporalen Blutkreislauf mindestens ein Ultraschallsensor angeordnet, der mit einer Auswerteeinheit verbunden ist. Zu Beginn der Filtration wird ein erstes Ultraschallsignal gespeichert und während der Filtration die Veränderung der Ultraschallsignale ermittelt. Aus der Änderung der Ultraschallsignale wird die Veränderung des Hämatokrits ermittelt und hieraus auf die Veränderung des intravasalen Blutvolumens geschlossen. Die Messung beruht dabei auf einem Zusammenhang zwischen der Schallgeschwindigkeit in dem Blut und dem jeweiligen Proteingehalt.

Aus der DE 44 19 593 A1 ist eine Vorrichtung zum nicht-invasiven Messen des Drucks eines Mediums bekannt. Die Vorrichtung besteht aus einer Druckmeßkammer, durch die beispielsweise Blut geleitet wird. Die Druckmeßkammer weist weiterhin einen Durchbruch auf, der durch eine Membran abgeschlossen ist. Die Membran ist mit einem Gummiring unterlegt, auf den ein Drucksensor aufgepresst wird, so dass sowohl positive als auch negative Drücke gemessen werden können.

Aus der EP 0 392 897 A2 ist ein Glasfibersensor bekannt, mit dem Druck, Temperatur und Durchfluss gemessen werden kann. Hierzu ist eine Glasfaser am Ende mit reflektiven und temperaturabhängigen Materialien abgeschlossen. Die reflektierte Lichtmenge ist dabei proportional zu dem Druck gegen das abgeschlossene Element.

Aus der EP 0 130 441 B1 ist eine Druckmeßvorrichtung bekannt, bei der ein Kontakt zwischen einer Druckkammer und einem Drucksensor dadurch sichergestellt wird, dass die Druckkammer gegen den Drucksensor angesaugt wird. Die angesaugte Fläche überträgt dabei Druckänderungen auf den Drucksensor.

Eine gattungsgemäße Meßvorrichtung ist bereits aus der EP-A-0374858 bekannt.

Bei der Dialysebehandlung zur Messung der oben genannten Parameter müssen mehrere getrennte Meßvorrichtungen vorgesehen werden, die zum Teil auch noch eigene Anbindungen an den extrakorporalen Kreislauf benötigen. Hierdurch entsteht ein erheblicher Meßaufwand.

Aufgabe der Erfindung ist es daher, eine Sensoreinrichtung zu schaffen, mit der die oben genannten Parameter in einem gemeinsamen Meßkopf gemessen werden können.

Die Aufgabe wird durch die Merkmale des Patentanspruchs 1 gelöst. Erfindungsgemäß besteht die Vorrichtung aus einer Meßkammer, durch die die zu messende Flüssigkeit geführt ist und die an einer Wandung mit einer flexiblen Membran abgeschlossen ist, und aus einer Meßplatte, die an ihrem äußeren Rand eine umlaufende Dichtung aufweist und die auf der flexiblen Membran aufliegt, wobei auf der Meßplatte ein Sensor zur Messung eines Parameters der medizinischen Flüssigkeit eingelassen ist und wobei die Meßplatte eine zur flexiblen Membran durchgehende Zuführung aufweist, über die zwischen der Meßplatte und der flexiblen Membran in Unterdruck aufbaubar ist. Erfindungsgemäß ist auf der Meßplatte mindestens ein weiterer Sensor aufgebracht, so dass unterschiedliche Meßfunktionen erfüllt werden können.

Die Erfindung nutzt die Erkenntnis, dass aufgrund der besonders ausgestalteten Meßplatte mit der daran anliegenden flexiblen Membran mehrere Sensoren auf der Meßplatte aufgebracht werden können. Aufgrund der umlaufenden Dichtung an der Meßplatte kann dabei die flexible Membran durch Anlegen des Unterdrucks in engem Kontakt mit der Unterseite der Meßplatte gebracht werden, so dass auch zwischen den Sensoren und der in der Meßkammer befindlichen Probe ein guter Meßkontakt herstellbar ist. Vorzugsweise schließt die Kontaktfläche der Sensoren mit dem unteren Rand der Meßplatte bündig ab. Auf diese Weise kann ein direkter Meßkontakt zwischen dem jeweiligen Sensor und der flexiblen Membran hergestellt werden.

Erfindungsgemäß können auf der Meßplatte mehrere Sensoren aufgebracht sein, so dass verschiedene Meßfunktionen erfüllt werden können. Aufgrund der fortschreitenden Integrationstechnik auch bei Sensoren ist es hierbei möglich, mehrere Sensoren auf einer wenige Quadratzentimeter großen Fläche anzuordnen. Der jeweilige Sensor wird dabei zweckmäßigerweise in eine Aussparung der Meßplatte eingelassen, wobei die Meßfläche des Sensors mit der Unterseite der Meßplatte bündig abschließt. Zur Fixierung des Sensors ist dabei dieser zweckmäßigerweise mit der Meßplatte verklebt. Als zu verwendende Sensoren kommen dabei insbesondere ein Drucksensor und ein Temperatursensor in Betracht. Drucksensoren sind durch die fortschreitende Integration auf dem Gebiet der Mikrosystemtechnik inzwischen auf einzelnen Halbleiterchips erhältlich, so dass diese eine Ausdehnung von wenigen Quadratmillimetern aufweisen. Da die Sensorfläche mit der Membran direkt in Berührung gebracht werden kann, ist die Messung von sowohl negativen als auch positiven Drücken möglich. Bei einem Dialysegerät mit einem Drucksensor und einem Temperatursensor kann damit sowohl die thermische Energiebilanz als auch der venöse Druck gemessen werden.

Nach einer weiteren Ausführungsform, für die selbstständiger Schutz beansprucht wird, ist eine weitere Meßplatte mit einer an ihrem Rand umlaufenden Dichtung vorgesehen, die gegenüber der ersten Meßplatte durch Abstandshalter parallel zueinander beabstandet ist, wobei dazwischen die Meßkammer eingebracht ist und die Wandung der Meßkammer gegenüber der weiteren Meßplatte ebenfalls mit einer flexiblen Membran abgeschlossen ist. Jede Meßplatte weist eine zur flexiblen Membran durchgehende Zuführung auf, über die zwischen der Meßplatte und der flexiblen Membran ein Unterdruck aufbaubar ist. Aufgrund der parallelen Meßplatten kann mit dieser Vorrichtung auch eine Ultraschall-Laufzeitmessung durchgeführt werden, wobei hierfür auf der ersten Meßplatte ein Ultraschallsensor eingelassen sein kann. Ein Ultraschallimpuls wird seitens des Ultraschallsensors ausgesendet, von der gegenüberliegenden Platte reflektiert und sodann von dem Ultraschallsensor wieder empfangen. Durch die Abstandhalter ist gewährleistet, dass sich der Abstand der beiden Meßplatten nicht ändert, so dass sehr genaue Ultraschall-Laufzeitmessungen durchgeführt werden können.

Die parallelen Meßplatten können weiterhin dazu benutzt werden, um Durchgangsmessungen an der Probe durchzuführen. Hierzu ist auf der einen Meßplatte mindestens ein Sendelement und auf der anderen Meßplatte mindestens ein dem Sendelement zugeordnetes Empfängerelement eingelassen. Das Sendeelement kann beispielsweise aus einem Ultraschallsender und/oder einer Lichtquelle bestehen, wobei das Empfängerelement in korrespondierender Weise aus einem Ultraschallempfänger und/oder einem Lichtempfänger auf der gegenüberliegenden Platte besteht.

Mit gegenüberliegenden Ultraschallsensoren kann das gemäß der oben genannten DE 38 27 553 C1 beschriebene Ultraschall-Meßverfahren zur Bestimmung des relativen Blutvolumens durchgeführt werden. Es sind weiterhin Meßvorrichtungen bekannt, bei denen zur Bestimmung der Ultraschallaufzeit eine Meßkammer aus Glas vorgesehen sein muß, um die Abstände der gegenüberliegenden Ultraschallelemente und damit auch die erforderliche Meßgenauigkeit einhalten zu können. Die erfindungsgemäße Vorrichtung ermöglicht es demgegenüber, auf eine aufwendige Meßkammer aus Glas zu verzichten.

Mit den gegenüberliegenden Ultraschallsensoren ist außerdem auch die Erkennung von Luftblasen in der jeweiligen Probe möglich.

Der optische Detektor bestehend aus Lichtquelle und Lichtempfänger kann beispielsweise für die automatische Bluterkennung oder auch für die Erkennung von Luftblasen verwendet werden.

Die Dichtung der Meßplatte besteht zweckmäßigerweise aus einem Gummiring, der in einer Nut der Meßplatte eingelassen ist und geringfügig über den Rand der Meßplatte hinausragt. Sobald zwischen der Membran und der Meßplatte ein Unterdruck aufgebaut wird, saugt sich die Membran an die Unterseite der Meßplatte fest, wobei durch die Dichtung gewährleistet ist, daß keine weitere Luft in den Bereich zwischen der Meßplatte und der flexiblen Membran nachströmt.

Die flexible Membran besteht vorzugsweise aus einer dünnen Plastikfolie, während die Meßkammer aus einer Plastikkammer mit starren Wänden bestehen kann. Besonders vorteilhaft ist es dabei, wenn die flexible Membran und die Meßkammer in einem Plastik-Einwegteil integriert sind. In dem Einwegteil können dabei Zentrierlöcher vorgesehen sein, durch die die Abstandhalter eingreifen, so daß das Einwegteil gegenüber den Meßplatten dauerhaft und sicher positioniert ist.

Die jeweilige Meßplatte besteht vorzugsweise aus einer Metallscheibe, in die die jeweiligen Sensoren eingelassen sind. Vorzugsweise wird die Metallscheibe dabei durch Peltierelemente auf einer konstanten Temperatur gehalten. Auf diese Weise ist eine genauere Temperaturmessung der jeweiligen Probe möglich.

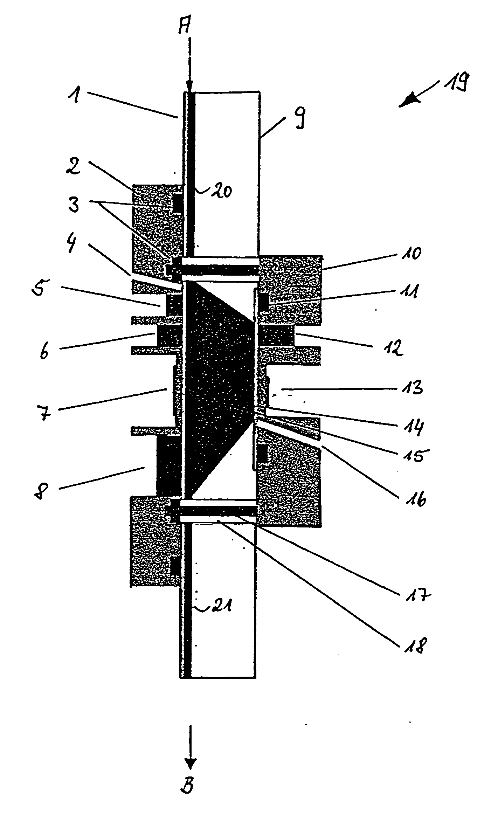

Weitere Einzelheiten und Vorteile werden anhand eines in der Zeichnung dargestellten Ausführungsbeispiels näher erläutert. Deren einzige Figur zeigt einen Schnitt durch die erfindungsgemäße Vorrichtung.

In einem Plastik-Einwegteil 9 ist eine Meßkammer 15 integriert, die einen Zufluß 20 und einen Abfluß 21 aufweist. An jeweils gegenüberliegenden Seiten ist die Meßkammer mit einer Folie 1, 14 abgeschlossen. Beidseitig der Meßkammer liegen an den Folien 1, 14 jeweils Meßplatten 2, 10 an, die an ihrem äußeren Rand jeweils eine Dichtung 3, 11 aufweisen. Die Dichtung ist dabei in einer jeweils dafür vorgesehenen Nut eingesetzt und ragt geringfügig über die Unterseite der Meßplatte hinaus. Gegeneinander sind die beiden Meßplatten über Abstandhalter 18 mit Schrauben 17 verschraubt.

An den Meßplatten sind jeweils Anschlüsse 4, 16 für Unterdruck vorgesehen. Durch Beaufschlagung mit Unterdruck werden dabei die Folien 1, 14 angesaugt, wobei die Dichtungen 3, 11 ein Nachströmen von Luft in den Raum zwischen Folie und Unterseite der Meßplatten verhindern. Auf diese Weise liegt die Meßkammer 15 beidseitig dicht abgeschlossen an den Meßplatten an.

In die Meßplatten sind mehrere Sensoren eingelassen, wobei die Meßplatten hierzu Durchgangsbohrungen oder entsprechende Aussparungen aufweisen. Die Sensoren sind jeweils in die Aussparungen eingesetzt und verklebt und schließen mit ihrer Unterseite dabei bündig mit der jeweiligen Unterseite der Meßplatte ab. Als Sensoren können beispielsweise ein Temperatursensor 5 und ein Drucksensor 8 vorgesehen sein, die einseitig seitens der Meßplatte 2 die Temperatur bzw. den Druck in der Meßkammer 15 messen. Aufgrund der dicht anliegenden Folie an der Unterseite des Drucksensors können dabei sowohl negative als auch positive Drücke gemessen werden. Sodann sind zusätzlich ein optischer Sensor sowie ein Ultraschallsensor für Durchgangsmessungen vorgesehen. Der optische Sensor besteht aus einer Lichtquelle 12 und einem Lichtempfänger 6, so daß hierdurch eine Lichtschranke gebildet wird. Der Ultraschallsensor besteht in entsprechender Weise aus einem Ultraschallsender 13 und einem Ultraschallempfänger 7.

Die Vorrichtung 19 ist vorzugsweise zur Dialysebehandlung vorgesehen. Hierzu wird die Vorrichtung 19 in den extrakorporalen Blutkreislauf verschaltet, so daß das Blut in Richtung der Pfeile A, B fließt. Vor den einzelnen Messungen wird zunächst an die Anschlüsse 4, 16 Unterdruck angelegt, so daß die Folien 1, 14 in gutem Kontakt zu den Sensoren stehen. Sodann werden die jeweiligen Sensoren über eine nicht näher dargestellte Steuereinheit angesteuert, so daß die jeweiligen Messungen beginnen können. Zur genaueren Temperaturmessung können dabei die Meßplatten durch Peltierelemente temperiert werden.

## Patentansprüche

1. Vorrichtung zur Messung von Parametern medizinischer Flüssigkeiten,
mit einer Meßkammer (15), durch die die zu messende Flüssigkeit geführt ist und die an einer Wandung mit einer flexiblen Membran (1) abgeschlossen ist,
mit einer Meßplatte (2), die an ihrem äußeren Rand eine umlaufende Dichtung (3) aufweist und die auf der flexiblen Membran (1) aufliegt,
mit einem Sensor (8) zur Messung eines Parameters der medizinischen Flüssigkeit, der in der Meßplatte (2) eingelassen ist, wobei die Meßplatte (2) eine zur flexiblen Membran (1) durchgehende Zuführung aufweist, über die zwischen der Meßplatte (2) und der flexiblen Membran (1) ein Unterdruck aufbaubar ist,
**dadurch gekennzeichnet,**
**dass** auf der Messplatte mindestens ein weiterer Sensor (5) mit einer unterschiedlichen Messfunktion aufgebracht ist.

2. Vorrichtung nach Anspruch 1, **dadurch gekennzeichnet, dass** ein Sensor ein Drucksensor (8) zur Messung des Drucks an der flexiblen Membran und/oder ein Temperatursensor (5) zur Messung der Temperatur an der flexiblen Membran ist.

3. Vorrichtung nach einem der Ansprüche 1 bis 2, **dadurch gekennzeichnet, dass** die Kontaktfläche der Sensoren mit dem unteren Rand der Meßplatte bündig abschließt.

4. Vorrichtung nach einem der Ansprüche 1 bis 3, **dadurch gekennzeichnet, dass** eine weitere Meßplatte (10) mit einer an ihrem Rand umlaufenden Dichtung (11) vorgesehen ist, die gegenüber der ersten Meßplatte (2) durch Abstandhalter parallel zueinander beabstandet ist, wobei dazwischen die Meßkammer eingebracht ist und die Wandung der Meßkammer gegenüber der weiteren Meßplatte (10) ebenfalls mit einer flexiblen Membran (14) abgeschlossen ist und dass die weitere Meßplatte (10) eine zur ihr zugeordneten flexiblen Membran (14) durchgehende Zuführung aufweist, über die zwischen Meßplatte (10) und der flexiblen Membran (14) ein Unterdruck aufbaubar ist.

5. Vorrichtung nach einem der Anspruch 4, **dadurch gekennzeichnet, dass** auf einer der beiden Meßplatten (2, 10) ein Ultraschallsensor (13) für eine Ultraschall-Laufzeitmessung eingelassen ist.

6. Vorrichtung nach einem der Ansprüche 4 bis 5, **dadurch gekennzeichnet, daß** auf der einen Meßplatte (2, 10) mindestens ein Sendeelement und auf der anderen Meßplatte (10, 2) mindestens ein dem Sendeelement zugeordnetes Empfängerelement eingelassen ist.

7. Vorrichtung nach Anspruch 6, **dadurch gekennzeichnet, dass** das Sendeelement ein Ultraschallsender (13) und/oder eine Lichtquelle (12) und das Empfängerelement ein Ultraschallempfänger (7) und/oder ein Lichtempfänger (6) ist.

8. Vorrichtung nach einem der Ansprüche 1 bis 7, **dadurch gekennzeichnet, dass** die Dichtung (3, 11) aus einem Gummiring besteht, der in einer Nut der Meßplatte eingelassen ist und geringfügig über den Rand der Meßplatte hinausragt.

9. Vorrichtung nach einem der Ansprüche 1 bis 8, **dadurch gekennzeichnet, dass** die flexible Membran aus einer Plastikfolie (1, 14) besteht.

10. Vorrichtung nach einem der Ansprüche 1 bis 9, **dadurch gekennzeichnet, dass** die Meßkammer aus einer Plastikkammer mit starren Wänden besteht.

11. Vorrichtung nach einem der Ansprüche 1 bis 10, **dadurch gekennzeichnet, dass** die flexible Membran (1, 14) und die Meßkammer in einem Einwegteil integriert sind.

12. Vorrichtung nach Anspruch 11, **dadurch gekennzeichnet, dass** die Abstandhalter in dafür vorgesehene Zentrierlöcher des Einwegteils eingreifen.

13. Vorrichtung nach einem der Ansprüche 1 bis 12, **dadurch gekennzeichnet, dass** die Meßplatte (2, 10) aus einer Metallscheibe besteht, in die die jeweiligen Sensoren (5, 8) eingelassen sind.

14. Vorrichtung nach Anspruch 13, **dadurch gekennzeichnet, dass** die Metallscheibe durch Peltierelemente auf einer konstanten Temperatur gehalten wird.

15. Vorrichtung nach einem der Ansprüche 1 bis 14, **dadurch gekennzeichnet, dass** die Sensoren (5, 8) jeweils mit einer Recheneinheit verschaltet sind.

## Claims

1. Apparatus for measuring parameters of medical fluids, comprising
a measurement chamber (15) through which the fluid to be measured is passed and which is closed off at a wall by a flexible diaphragm (1),
a measurement plate (2) which at its outer edge has a peripherally extending seal (3) and which bears against the flexible diaphragm (1), and
a sensor (8) for measuring a parameter of the medical fluid, which is fitted in the measurement plate (2), wherein the measurement plate has a feed through to the flexible diaphragm (1) and by way of which a reduced pressure can be built up between the measurement plate (2) and the flexible diaphragm (1),
**characterised in that**
at least one further sensor (5) with a different measurement function is disposed on the measurement plate.

2. Apparatus according to claim 1 **characterised in that** a sensor is a pressure sensor (8) for measuring the pressure at the flexible diaphragm and/or a temperature sensor (5) for measuring the temperature at the flexible diaphragm.

3. Apparatus according to one of claims 1 to 2 **characterised in that** the contact surface of the sensors terminates flush with the lower edge of the measurement plate.

4. Apparatus according to one of claims 1 to 3 **characterised in that** there is provided a further measurement plate (10) having a peripherally extending seal (11) at its edge, which is spaced with respect to the first measurement plate (2) by spacers in mutually parallel relationship, wherein the measurement chamber is disposed therebetween and the wall of the measurement chamber is also closed off with respect to the further measurement plate (10) with a flexible diaphragm (14) and that the further measurement plate (10) has a through feed to the flexible diaphragm (14) associated therewith, by way of which feed a reduced pressure can be built up between the measurement plate (10) and the flexible diaphragm (14).

5. Apparatus according to claim 4 **characterised in that** an ultrasonic sensor (13) for ultrasound transit time measurement is fitted on one of the two measurement plates (2, 10).

6. Apparatus according to one of claims 4 and 5 **characterised in that** at least one transmitter element is fitted on the one measurement plate (2, 10) and at least one receiver element associated with the transmitter element is fitted on the other measurement plate (10, 2).

7. Apparatus according to claim 6 **characterised in that** the transmitter element is an ultrasonic transmitter (13) and/or a light source (12) and the receiver element is an ultrasonic receiver (7) and/or a light receiver (6).

8. Apparatus according to one of claims 1 to 7 **characterised in that** the seal (3, 11) comprises a rubber ring which is fitted in a groove in the measurement plate and which projects slightly beyond the edge of the measurement plate.

9. Apparatus according to one of claims 1 to 8 **characterised in that** the flexible diaphragm is a plastic film (1, 14).

10. Apparatus according to claims 1 to 9 **characterised in that** the measurement chamber comprises a plastic chamber with rigid walls.

11. Apparatus according to one of claims 1 to 10 **characterised in that** the flexible diaphragm (1, 14) and the measurement chamber are integrated in a disposable part.

12. Apparatus according to claim 11 **characterised in that** the spacers engage into centering holes provided for that purpose in the disposable part.

13. Apparatus according to one of claims 1 to 12 **characterised in that** the measurement plate (2, 10) comprises a metal disc into which the respective sensors (5, 8) are let.

14. Apparatus according to claim 13 **characterised in that** the metal disc is held at a constant temperature by Peltier elements.

15. Apparatus according to one of claims 1 to 14 **characterised in that** the sensors (5, 8) are respectively connected to a computing unit.

## Revendications

1. Dispositif pour la mesure de paramètres de liquides médicinaux,
avec une enceinte de mesure (15) à travers laquelle est guidé le liquide à mesurer et qui est fermée à une paroi par une membrane flexible (1),
avec une plaque de mesure (2) qui présente à son bord extérieur une garniture d'étanchéité s'étendant tout autour (3) et qui repose sur la membrane flexible (1),
avec un capteur (8) pour mesurer un paramètre du liquide médicinal, qui est inséré dans la plaque de mesure (2), où la plaque de mesure (2) présente une amenée traversante à la membrane flexible (1), par laquelle une dépression peut être formée entre la plaque de mesure (2) et la membrane flexible (1)
**caractérisé en ce qu'**est monté sur la plaque de mesure au moins un autre capteur (5) avec une fonction de mesure différente.

2. Dispositif selon la revendication 1, **caractérisé en ce qu'**un capteur est un capteur de pression (8) pour mesurer la pression à la membrane flexible et/ou un capteur de température (5) pour mesurer la température à la membrane flexible.

3. Dispositif selon l'une des revendications 1 à 2, **caractérisé en ce que** la face de contact des capteurs se termine en affleurement avec le bord inférieur de la plaque de mesure.

4. Dispositif selon l'une des revendications 1 à 3, **caractérisé en ce qu'**une plaque de mesure supplémentaire (10) avec une garniture d'étanchéité (11) s'étendant à son bord est prévue qui est espacée parallèlement par rapport à la première plaque de mesure (2) par des pièces d'écartement, où est disposée entre celles-ci l'enceinte de mesure, et la paroi de l'enceinte de mesure est fermée par rapport à l'autre plaque de mesure (10) également par une membrane flexible (14), et **en ce que** la plaque de mesure supplémentaire (10) présente une amenée traversante à la membrane flexible (14) associée à celle-ci par laquelle une dépression peut être créée entre la plaque de mesure (10) et la membrane flexible (14).

5. Dispositif selon la revendication 4, **caractérisé en ce que** sur l'une des deux plaques de mesure (2, 10) est inséré un capteur ultrasonore (13) pour une mesure de la durée d'exécution ultrasonore.

6. Dispositif selon l'une des revendications 4 à 5, **caractérisé en ce qu'**est monté sur une plaque de mesure (2, 10) au moins un élément émetteur et sur l'autre plaque de mesure (10, 2) au moins un élément récepteur associé à l'élément émetteur.

7. Dispositif selon la revendication 6, **caractérisé en ce que** l'élément émetteur est un émetteur ultrasonore (13) et/ou une source de lumière (12), et l'élément récepteur est un récepteur ultrasonore (7) et/ou un récepteur de lumière (6).

8. Dispositif selon l'une des revendications 1 à 7, **caractérisé en ce que** la garniture d'étanchéité (3, 11) est constituée d'une bague en caoutchouc qui est insérée dans une rainure de la plaque de mesure et qui fait légèrement saillie sur le bord de la plaque de mesure.

9. Dispositif selon l'une des revendications 1 à 8, **caractérisé en ce que** la membrane flexible est constituée d'une feuille plastique (1, 14).

10. Dispositif selon l'une des revendications 1 à 9, **caractérisé en ce que** l'enceinte de mesure est constituée d'une enceinte plastique avec des parois rigides.

11. Dispositif selon l'une des revendications 1 à 10, **caractérisé en ce que** la membrane flexible (1, 14) et l'enceinte de mesure sont intégrées dans une pièce à usage unique.

12. Dispositif selon la revendication 11, **caractérisé en ce que** les pièces d'écartement s'engagent dans des trous de centrage prévus à cette fin de la pièce à usage unique.

13. Dispositif selon l'une des revendications 1 à 12, **caractérisé en ce que** la plaque de mesure (2, 10) est constituée d'un disque métallique dans lequel sont insérés les capteurs respectifs (5, 8).

14. Dispositif selon la revendication 13, **caractérisé en ce que** le disque métallique est maintenu par des éléments de Peltier à une température constante.

15. Dispositif selon l'une des revendications 1 à 14, **caractérisé en ce que** les capteurs (5, 8) sont connectés chacun à une unité de calcul.
